(19) Europäisches Patentamt
European Patent Office
Office européen des brevets

(11) EP 0 755 928 B1

(12) **EUROPEAN PATENT SPECIFICATION**

(45) Date of publication and mention of the grant of the patent:
**10.04.2002 Bulletin 2002/15**

(21) Application number: **96901492.7**

(22) Date of filing: **31.01.1996**

(51) Int Cl.[7]: **C07D 311/30**, A61K 31/35

(86) International application number:
**PCT/JP96/00181**

(87) International publication number:
**WO 96/24592 (15.08.1996 Gazette 1996/37)**

(54) **5-AMINOFLAVONE DERIVATIVES**
5-AMINOFLAVON-DERIVATE
DERIVES 5-AMINOFLAVONE

(84) Designated Contracting States:
**DE FR GB IT**

(30) Priority: **06.02.1995 JP 1774195**

(43) Date of publication of application:
**29.01.1997 Bulletin 1997/05**

(73) Proprietor: **KYOWA HAKKO KOGYO CO., Ltd.**
**Chiyoda-ku, Tokyo 100 (JP)**

(72) Inventors:
- **AKAMA, Tsutomu**
  **Tokyo 194 (JP)**
- **IKEDA, Shun-ichi**
  **Tokyo 194 (JP)**
- **ISHIDA, Hiroyuki Oakwood Apartments D205**
  **Mountain View, CA 94043 (US)**
- **KIMURA, Uichiro**
  **Fukuoka 810 (JP)**
- **GOMI, Katsushige**
  **Shizuoka 410-11 (JP)**
- **SAITO, Hiromitsu**
  **Kanagawa 214 (JP)**

(74) Representative: **VOSSIUS & PARTNER**
**Siebertstrasse 4**
**81675 München (DE)**

(56) References cited:
**JP-A- 2 138 277**
**JP-A- 2 256 673**
**JP-A- 5 286 962**
**JP-A- 7 109 268**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 755 928 B1

Printed by Jouve, 75001 PARIS (FR)

## Description

### Field of the Invention

The present invention relates to novel 5-aminoflavone derivatives possessing anti-bacterial activity, anti-estrogenic activity, and antitumor activity.

### Background of the Invention

As derivatives having an amino group at 5-position and a fluorine atom at 8-position of flavone (2-phenyl-4H-1-benzopyran-4-one), there are disclosed compounds possessing a nti-cellular activity [Chem. Absts., 113, 171775n (1990)]. However, the compounds do not have an amino group at 4'-position and no embodiments thereof are disclosed. As derivatives having amino groups at 5-position and 4'-position, there are disclosed compounds possessing anti-cellular activity (EP-A-374789). However, the compounds have no fluorine atom at 6-position and 8-position and have no substituents at 7-position, either. As derivatives having amino groups at 5-position and 4'-position, and fluorine atoms at 6-position and 8-position, there are disclosed compounds possessing anti-cellular activity (EP556720). However, the compounds have no substituents at 7-position.

As other derivatives having amino groups at 5-position, there are disclosed compounds having a hydroxyl group at 6-position [Chem. Abst., 120f (1947)], compounds having an alkoxy group at 3-position with antiviral activity (EP-A-23105), compounds having anti-allergy activity and the like (GB-A-1461777) and compounds having a methyl group at 7-position [Arch. Pharm. (Weinheim), 322, 589 (1989)], and compounds having amino groups at 5-position, 7-position, and 4'-position with inhibitory activity against tyrosine-kinase [J. Med. Chem., 37, 3353 (1994)]. Further, there are disclosed derivatives having halogen atoms at 6- and/or 8-position and an amino group at 4'-position [Indian J. Chem., 1, 477, (1963)]. However, anti-cellular activity of the above compounds is not known.

Further, anti-estrogenic activity is not known in respect of the flavone derivatives described above.

5-Aminoflavone derivatives having anti-bacterial, anti-estrogenic and/or anti-tumor activity as well as their preparation have been disclosed in EP-0 638 566 A1.

### Disclosure of the Invention

The present invention provides a 5-aminoflavone derivative represented by the formula (I) :

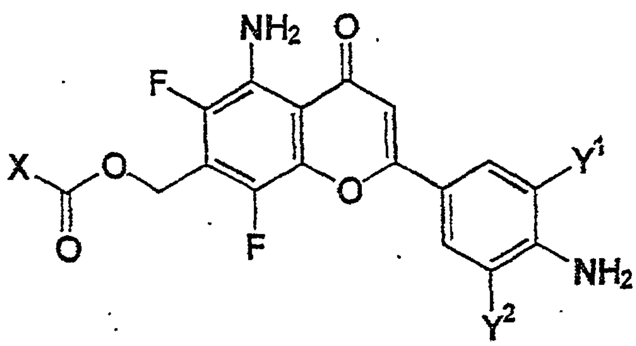

(I)

wherein X represents (i) $C_1$-$C_6$ alkyl substituted with 1 to 3 substituents selected from a group consisting of $NR^3R^4$ {wherein $R^3$ and $R^4$ are the same or different and represent hydrogen, $C_1$-$C_6$ alkyl which may be substituted with $NR^5R^6$ (wherein $R^5$ and $R^6$ are the same or different and represent hydrogen, or $C_1$-$C_6$ alkyl), or $R^3$ and $R^4$ are taken together to form a heterocyclic group containing the sandwiched nitrogen atom in the ring which may be substituted with 1 to 3 substituents selected from a group consisting of $C_1$-$C_6$ alkyl and $NR^7R^8$ (wherein $R^7$ and $R^8$ are the same or different and represent hydrogen, or $C_1$-$C_6$ alkyl)}, halogen, hydroxy, $C_1$-$C_6$ alkoxy, $C_1$-$C_6$ alkoxycarbonyl, $C_1$-$C_6$ alkanoyl, $C_1$-$C_6$ alkanoyloxy, and vinyl, (ii) $C_1$-$C_6$ alkoxy which may be substituted with 1 to 3 substituents selected from a group consisting of $NR^3R^4$ {wherein $R^3$ and $R^4$ are the same or different and represent hydrogen, $C_1$-$C_6$ alkyl which may be substituted with $NR^5R^6$ (wherein $R^5$ and $R^6$ are the same or different and represent hydrogen, or $C_1$-$C_6$ alkyl), or $R^3$ and $R^4$ are taken together to form a heterocyclic group containing the sandwiched nitrogen atom in the ring which may be substituted with 1 to 3 substituents selected from a group consisting of $C_1$-$C_6$ alkyl and $NR^7R^8$ (wherein $R^7$ and $R^8$

are the same or different and represent hydrogen, or $C_1$-$C_6$ alkyl)}, halogen, hydroxy, $C_1$-$C_6$ alkoxy, $C_1$-$C_6$ alkoxycarbonyl, $C_1$-$C_6$ alkanoyl, $C_1$-$C_6$ alkanoyloxy, and vinyl, or (iii) $NR^1R^2$ <wherein $R^1$ and $R^2$ are the same or different and represent hydrogen, $C_1$-$C_6$ alkyl which may be substituted with 1 to 3 substituents selected from a group consisting of $NR^3R^4$ {wherein $R^3$ and $R^4$ are the same or different and represent hydrogen, $C_1$-$C_6$ alkyl which may be substituted with $NR^5R^6$ (wherein $R^5$ and $R^6$ are the same or different and represent hydrogen, or $C_1$-$C_6$ alkyl), or $R^3$ and $R^4$ are taken together to form a heterocyclic group containing the sandwiched nitrogen atom in the ring which may be substituted with 1 to 3 substituents selected from a group consisting of $C_1$-$C_6$ alkyl and $NR^7R^8$ (wherein $R^7$ and $R^8$ are the same or different and represent hydrogen, or $C_1$-$C_6$ alkyl)}, halogen, hydroxy, $C_1$-$C_6$ alkoxy, $C_1$-$C_6$ alkoxycarbonyl, $C_1$-$C_6$ alkanoyl, $C_1$-$C_6$ alkanoyloxy, and vinyl, or $R^1$ and $R^2$ are taken together to form a heterocyclic group containing the sandwiched nitrogen atom in the ring which may be substituted with 1 to 3 substituents selected from a group consisting of $C_1$-$C_6$ alkyl and $NR^7R^8$ (wherein $R^7$ and $R^8$ are the same or different and represent hydrogen, or $C_1$-$C_6$ alkyl)>, and

$Y^1$ and $Y^2$ are the same or different and represent hydrogen, halogen, or $C_1$-$C_6$ alkyl, or
a pharmaceutically acceptable salt thereof.

As pharmaceutically acceptable salts of Compound (I), there are pharmaceutically acceptable acid or base addition salts, for example, inorganic acid salts such as hydrochloride, hydrobromide, sulfate, phosphate, and the like, and organic acid salts such as methanesulfonate, oxalate, acetate, mabonate, succinate, fumarate, maleate, tartrate, citrate, and the like as well as base addition salts such as sodium salt, potassium salt, and the like.

Then, a process for producing Compound (I) is explained below.

In addition, in the process described below, when the defined groups may be changed under the process conditions or are not suitable for the process, such inconvenience can be avoided by a method usually used in organic synthetic chemistry, for example, protection and deprotection of functional groups and the like.

Process 1

Compound (Ia) which is Compound (I) wherein X is substituted lower alkyl can be prepared according to the following scheme:

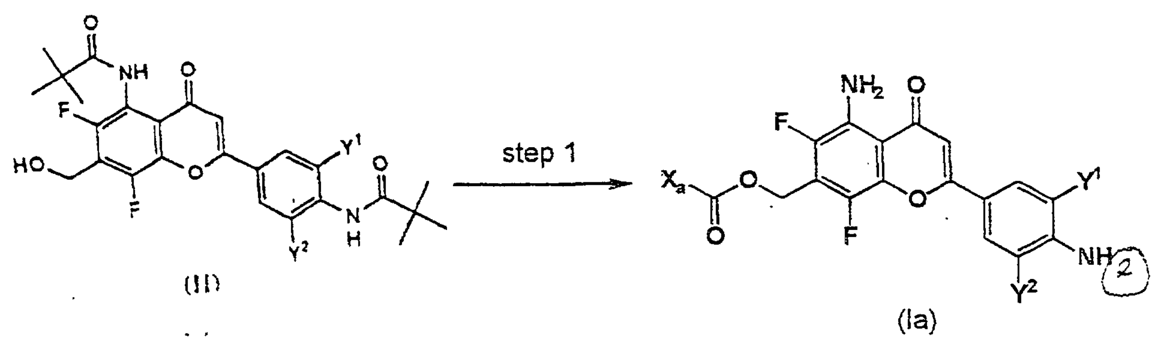


(wherein $X_a$ represents substituted lower alkyl in the definition of X, and $Y^1$ and $Y^2$ are the same as defined).

Step 1

Compound (Ia) is prepared by simultaneous removing of the pivaloyl group and esterification in Compound (II), prepared by Reference Example 1 or modified method thereof, under heating in the presence of conc. sulfuric acid with the solvent of the carboxylic acid corresponding to the substituted lower alkanoyl moiety. The sulfuric acid is used preferably in concentration from 1 to 18 normality. The reaction is normally carried out under from room temperature to the boiling point of the corresponding solvent, preferably from 50 to 100 °C. The reaction completes in 0.1 to 10 hours.

Process 2

Compound (Ib) which is Compound (I) wherein X is unsubstituted or substituted lower alkoxy can be prepared according to the following scheme:

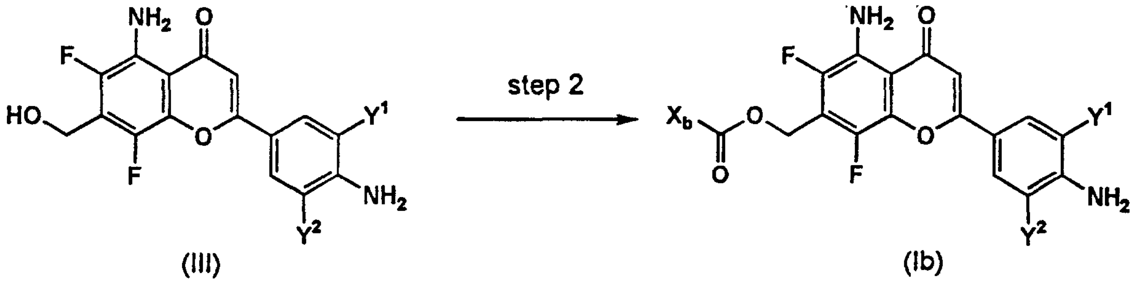

(III) (Ib)

(wherein $X_b$ represents unsubstituted or substituted lower alkoxy in the definition of X, and $Y^1$ and $Y^2$ are the same as defined)

Step 2

Compound (Ib) can be obtained by the reaction of (III) prepared by Reference Example 2 or modified method thereof, if necessary, in the presence of 0.1 to 1 equivalent of dimethylaminopyridine with 1 to 5 equivalents of the compound represented by $(X_bCO)_2O$. The reaction is carried out at 0 °C to room temperature and completes in 1 to 10 hours.

Process 3

Compound (Ic) which is Compound (I) wherein X is $NR^1R^2$ (wherein $R^1$ and $R^2$ are the same as defined) can be prepared according to the following scheme.

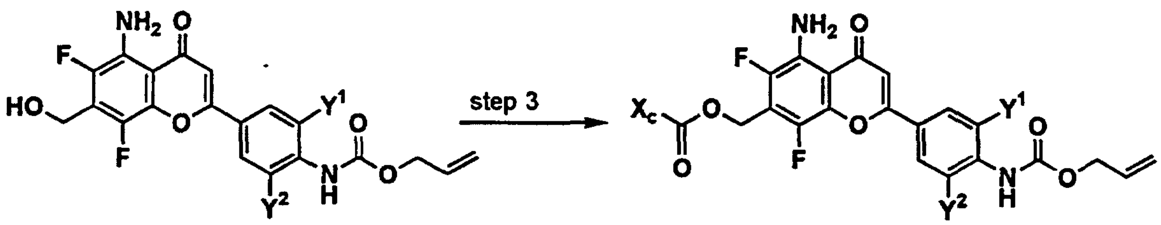

(IV) (Vc)

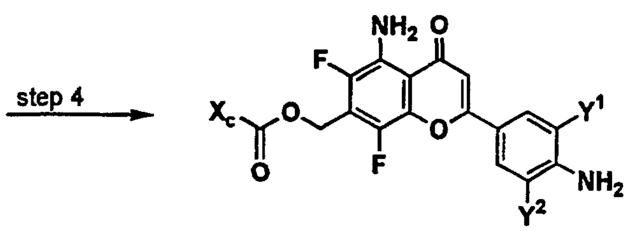

(Ic)

[wherein $X_c$ represents $NR^1R^2$ (wherein $R^1$ and $R^2$ are the same as defined) in the definition of X, and $Y^1$ and $Y^2$ are the same as defined].

Step 3

The reaction of Compound (IV) prepared by Reference Example 3 or modified method thereof with 1 to 5 equivalents of p-nitrophenyl chloroformate in a solvent in the presence of 2 to 5 equivalents of base affords the carbonate, which can be converted to carbamoyl group by adding the amine corresponding to $X_c$ moiety to give Compound

(Vc). As solvents, dichloromethane, dichloroethane, dimethylformamide, and the like are used. As bases, triethylamine, diisopropylamine, diisopropylethylamine, pyridine, dimethylaminopyridine, and the like are used. The reaction is carried out at 0 °C to room temperature, and completes in 1 to 10 hours.

Step 4

Compound (Ic) can be obtained by treating Compound (Vc), prepared by step 3, in a solvent, for example, tetrahydrofurane and the like, with 1 to 10 equivalents of formic acid-triethylamine in the presence of 0.01 to 0.2 equivalent of palladium tetrakistriphenylphosphine. The reaction is carried out at 0 °C to room temperature, and completes in 1 to 10 hours.
In addition, Compound (Ia) can also be prepared according to the following reaction scheme.

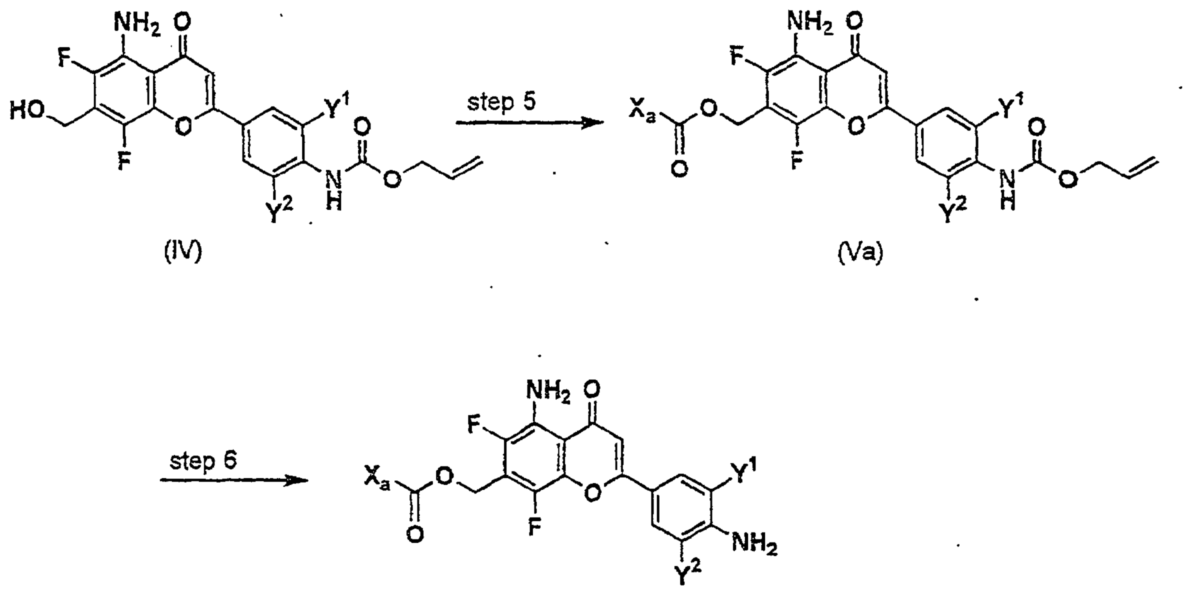


(IV) (Va)

(Ia)

(wherein $X_a$, $Y^1$, and $Y^2$ are the same as defined)

Step 5

Compound (Va) can be obtained by the reaction of Compound (IV) prepared by Reference Example 3 or modified method thereof, with 1 to 20 equivalents of the carboxylic acid or its activated derivative corresponding to substituted lower alkanoyl moiety in Compound (Ia) in an inert solvent in the presence of 1 to 20 equivalents of a condensation reagent or a base. As activated derivatives of a carboxylic acid, acid halide, acid anhydride, several activated ester, and the like are used. As inert solvents, dichloromethane, dichloroethane, toluene, dimethylformamide and the like are used. As condensation reagents, carbonyl diimidazole, dicyclohexylcarbodiimide, 2-chloro-N-methylpyridinium iodide, and the like are used. As bases, tertiary amine such as pyridine, triethylamine, etc., potassium carbonate, sodium carbonate, sodium hydride, and the like are used. The reaction is normally carried out under from 0 °C to the boiling point of the used solvent, preferably from room temperature to 80 °C, and completes in 1 hour to 1 week.

Step 6

Compound (Ia) can be obtained by treating Compound (Va) prepared by step 5 according to a modified method of the method of step 4 above.
The resulting Compound (Ia) can partly be used as the synthetic intermediate to be converted to further novel derivatives.
For example, Compound (Iaa), wherein $X_a$ is $(CH_2)_nNR^3R^4$ (wherein n is an integer of 1 to 6, and $R^3$ and $R^4$ are the same as defined) in Compound (Ia) can be obtained by the reaction of Compound (Iab), wherein $X_a$ is $(CH_2)_nY$ (Y represents chlorine, bromine, and iodine) with 1 to 10 equivalents of $HNR^3R^4$ (wherein $R^3$ and $R^4$ are the same as

defined above) in a inert solvent, if necessary, in the presence of base. As inert solvents, dimethylformamide, dimethylsulfoxide, tetrahydrofuran, dioxane, and the like are used. As bases, triethylamine, diisopropylethylamine, pyridine, dimethylaminopyridine, potassium carbonate, sodium carbonate, and the like are used. The reaction is carried out under from 0 to 100 °C, preferably from 50 to 70 °C, and completes in 1 to 10 hours.

Intermediates and desired compounds in the above processes can be isolated and purified by purifying methods normally used in organic synthetic chemistry, for example, filtration, extraction, washing, drying, concentration, recrystallization, and various chromatographies and the like. In addition, intermediates may also be subjected to the subsequent step without purification.

When a salt form of the product is desired, a salt compound can be subjected to known purification or isolation processes to give a salt form. When a product is synthesized in free form, a salt for Compound (I) can be separated or purified after formation of salt by normal method, for example, dissolving or suspending it in an appropriate organic solvent and adding an appropriate acid or base.

Compound (I) and a salt thereof can also be present in the form of addition products to water or various solvents. Such addition products are also included within the scope of the present invention.

Particular examples of Compound (I) of the present invention are shown in Table 1.

Table 1

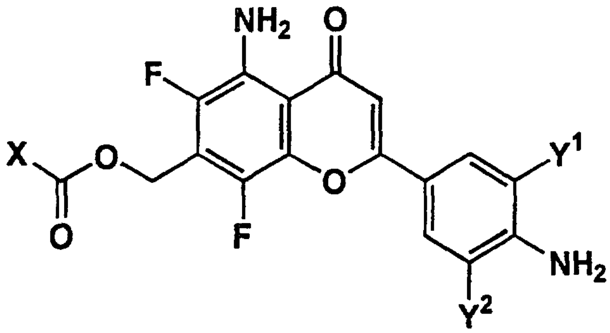

| compound | X | $Y^1$ | $Y^2$ |
|---|---|---|---|
| 1 | $N(CH_2CH_3)_2$ | F | H |
| 2 | 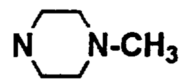 | F | H |
| 3 | $NH(CH_2)_2N(CH_3)_2$ | F | H |
| 4 | $CH_2Cl$ | F | H |
| 5 | $(CH_2)_2Br$ | F | H |
| 6 | $CH_2N(CH_3)_2$ | F | H |
| 7 | $(CH_2)_2N(CH_3)_2$ | F | H |
| 8 | $(CH_2)_3N(CH_3)_2$ | F | H |
| 9 | $CH_2$ 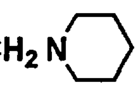 | F | H |
| 10 | $CH_2$ 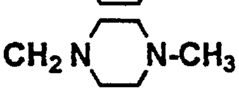 | F | H |
| 11 | $CH_2$ 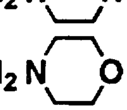 | F | H |
| 12 | $CH_2$ 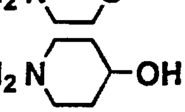 | F | H |
| 13 | $CH_2N(CH_3)(CH_2)_2N(CH_3)_2$ | F | H |
| 14 | $CH_2NHCH_2COOCH_3$ | F | H |
| 15 | $OCH_2CH=CH_2$ | F | H |
| 16 | $CH_2Cl$ | Cl | Cl |
| 17 | $CH_2N(CH_3)_2$ | Cl | Cl |

Compound (I) has the anti-estrogenic activity, which can be demonstrated by the decrease in uterus weight of a mature mouse. In addition, Compound (I) inhibits the growth of human mammary cancer cells in the medium of a microplate and inhibits the growth of human mammary cancers transplanted into nude mice.

The compound possessing such the bioactivity is useful for treatment of the symptoms for which tamoxifen

is useful, for example, breast cancer, non-ovulatory sterility, and paramenia.

Then, the actitity of the representative compounds of Compound (I) is shown by the following Test Examples.

Test Example 1

The effect against the decrease in uterus weight of mature mouse

Mature female BALB/c mice, 9 weeks age, were divided into two groups (6 animals per group). The test compounds were repeatedly administered orally to one group for 4 days. After 5 days, the uteri were isolated and the weight thereof was measured. The results are shown in Table 2.

Table 2

| Compound | dose (mg/kg) | weight of uterus (mg) |
|---|---|---|
| not treated | - | 106 |
| 6 | 25 | 67 |

Test Example 2

Human mammary cancer MCF-7 cell growth inhibition test

Each 0.1 ml of MCF-7 cells which had been prepared in a concentration of $5 \times 10^4$/ml using a medium prepared by adding 10% bovine fetal serum, $10^{-8}$M estradiol (manufactured by Sigma), 100 units/ml penicillin and 100 μg/ml streptomycin to RPMI1640 medium (referred to as Medium B hereinafter) was distributed in each well of 96 well-microtiter plate. The plate was allowed to stand at 37 °C for 20 hours in a $CO_2$ gas incubator, each 0.05 ml of samples (test compound) which had been appropriately diluted with Medium B was added thereto and the mixture was allowed to stand at 37 °C for 72 hours in a $CO_2$ gas incubator. The supernatant was removed, each 0.1 ml of Medium B containing 0.02% neutral red was added to the residue, the mixture was allowed to stand at 37 °C for 1 hour in a $CO_2$ gas incubator and the cells were stained by neutral red dye. The supernatant was removed and the residue was washed once with a physiological saline. Then, the dye was extracted with 0.001 N hydrochloric acid/30% ethanol and the absorbance at 550 nm was determined with a microplatereader. The concentration ($IC_{50}$) at which the growth of cell is inhibited by 50% was calculated by comparing the absorbance of non-treated cells and sample-treated cells. The results are shown in Table 3.

Table 3

| Compound | $IC_{50}$ (μM) |
|---|---|
| 1 | 0.35 |
| 2 | 0.23 |
| 6 | 0.026 |
| 7 | 0.019 |
| 8 | 0.029 |
| 9 | 0.033 |
| 10 | 0.087 |
| 11 | 0.017 |
| 12 | 0.018 |
| 14 | 0.013 |
| 15 | 0.081 |

Test Example 3

Antitumor effects against estrogen-dependent human mammary cancer MCF-7

The tumor fragment (2 mm x 2 mm x 2mm) of human hormone dependent mammary cancer MCF-7 was transplanted subcutaneously in the flank of female BALB/c-nu/nu mouse (Nihon Crea), 7 to 9 weeks age. For promoting the growth of tumor, 12.5 μg of estradiol propionate was intramuscularly administered in the femoral region two times in total, i.e., on the date of transplantation and two weeks after transplantation. Mice having the tumor volume 25 to

200 $mm^3$ were selected 3 to 4 weeks after transplantation, and the test compounds were orally administered repeatedly to the groups (5 animals per group) for 5 days per a week, for total two weeks. In addition, estradiol propionate was administered again on the date of initial administration of the test compounds. Length and width of the tumor were determined every day, and the tumor volumes were calculated by means of ellipsoid approximation according to the following equation:

$$\text{Tumor volume (mm)}^3 = [\text{Length (mm) x [width]}^2]/2$$

The tumor volume at initial administration ($V_0$) and on the day of judgement (V) was calculated, and the tumor growth rate ($V/V_0$) was calculated. T/C value was obtained as the ratio of $V/V_0$ value of treated group relative to that of control group. The results are shown in Table 4.

Table 4

| Compound | dose (mg/kg) | T/C | judgement date (day) |
|---|---|---|---|
| 6 | 25 | 0.012 | 15, 17 |
| 7 | 25 | 0.041 | 18 |
| 8 | 25 | 0.15 | 14 |
| 9 | 25 | 0.042 | 14 |
| 10 | 25 | 0.070 | 14 |
| 11 | 25 | 0.044 | 18 |
| 12 | 25 | 0.035 | 18 |
| 13 | 25 | 0.022 | 24 |
| 14 | 25 | 0.013 | 25 |

Test Example 4

Antibacterial activity

Antibacterial activity of Compound (I) against Bacillus subtilis #10107 [Minimum Inhibition Concentration (MIC; μg/ml)] is shown in Table 5. Minimum Inhibition Concentration was determined by agar dilution method at pH 7.0.

Table 5

| Compound | MIC (μM) |
|---|---|
| 3 | 83.3 |
| 6 | 26.0 |
| 7 | 104 |
| 8 | 104 |

The Examples and Reference Examples are shown below. Physicochemical data on the compounds in the following Examples and Reference Examples were determined using the following apparatus:

| | |
|---|---|
| $^1$H-NMR | JEOL JNM-GX270 (270MHz) |
| | JEOL JNM-EX270 (270MHz) |
| | HITACHI R-90H (90MHz) |
| MS | JEOL JMS-D300 |
| | JEOL JMS-SX102 |
| | SHIMAZU QP-1000 |

Example 1

5-Amino-2-(4-amino-3-fluorophenyl)-7-diethylcarbamoyloxymethyl-6, 8-difluoro-4H-1-benzopyran-4-one (compound 1)

(1) Compound (IVa) (303 mg, 0.722 mmol) obtained in Reference Example 3 was dissolved in dimethylformamide (15 mL). Triethylamine (0.3 mL, 2.2 mmol) and P-nitrophenyl chloroformate (293 mg, 1.44 mmol) were added to the mixture under ice-cooling and the mixture was stirred at room temperature overnight. The reaction solution was cooled on ice, diethylamine (0.75 mL, 7.2 mmol) was added and the mixture was stirred at the same temperature for 4.5 hours. Water was added to the reaction solution and the mixture was extracted with ethyl acetate. The organic layer was washed with water and brine and dried over anhydrous sodium sulfate, and the solvent was distilled off under reduced pressure. The residue was purified by silica gel column chromatography (chloroform), to give 2-(4-allyloxycarbonylamino-3-fluorophenyl)-5-amino-7-diethylcarbamoyloxymethyl-6,8-difluoro-4H-1-benzopyran-4-one (239 mg, yield: 64%).
$^1$H NMR (90 MHz, $CDCl_3$) δ(ppm) 1.12 (t, J = 7.3 Hz, 6H), 3.20 (q, J = 7.3 Hz, 2H), 3.28 (q, J = 7.3 Hz, 2H), 4.72 (d, J = 5.7 Hz, 2H), 5.2-5.5(m, 2H), 5.32 (s, 2H), 5.7-6.2 (m, 1H), 6.23 (brs, 2H), 6.57 (s, 1H), 7.09 (d, J = 3.3 Hz, 1H), 7.5-7.8 (m, 2H), 8.32 (t, J = 8.5Hz, 1H)
FABMS (m/e) 520 $(M+H)^+$ molecular formula $C_{25}H_{24}F_3N_3O_6$ = 519
(2) The above 2-(4-allyloxycarbonylamino-3-fluorophenyl)-5-amino-7-diethylcarbamoyloxymethyl-6,8-difluoro-4H-1-benzopyran-4-one (223 mg, 0.429 mmol) was dissolved in tetrahydrofuran (20 mL). Formic acid-triethylamine (0.29 mL) and tetrakis(triphenylphosphine)palladium (50 mg, 0.043 mmol) were added to the solution and the mixture was stirred at room temperature for 40 minutes. Water was added to the reaction solution and the mixture was extracted with ethyl acetate. The organic layer was washed with water and brine and dried over anhydrous sodium sulfate, and the solvent was distilled off under reduced pressure. The residue was purified by silica gel column chromatography (chloroform) and recrystallized from ethyl acetate/n-hexane, to give compound 1 (105 mg, yield: 56%).
$^1$H NMR (270 MHz, $CDCl_3$) δ(ppm) 1.12 (brs, 6H), 3.2-3.4 (m, 4H), 5.30 (s, 2H), 6.58 (s, 1H), 6.83 (t, J = 8.9 Hz, 1H), 7.5-7.6 (m, 2H)
FABMS (m/e) 436 $(M+H)^+$ molecular formula $C_{21}H_{20}F_3N_3O_4$ = 435

Example 2

5-Amino-2-(4-amino-3-fluorophenyl)-6,8-difluoro-7-(4-methylpiperazinyl)carbonyloxymethyl-4H-1-benzopyran-4-one (compound 2)

(1) Substantially the same manner as that in Example 1 (1) was repeated except that Compound (IVa) (420 mg, 1.00 mmol) obtained in Reference Example 3 was used and N-methylpiperazine (0.35 mL, 3.0 mmol) was used instead of diethylamine, to give 2-(4-allyloxycarbonylamino-3-fluorophenyl)-5-amino-6,8-difluoro-7-(4-methylpiperazinyl)carbonyloxymethyl-4H-1-benzopyran-4-one (294 mg, yield: 54%).
$^1$H NMR (90 MHz, $CDCl_3$) δ (ppm) 2.31 (s, 3H), 2.38 (t, J = 5.3 Hz, 4H), 3.52 (t, J = 5.1 Hz, 4H), 4.72 (d, J = 5.5 Hz, 2H), 5.2-5.5 (m, 2H), 5.33 (s, 2H), 5.7-6.2 (m, 1H), 6.23 (brs, 1H), 6.56 (s, 2H), 7.15 (d, J = 3.1 Hz, 1H), 7.5-7.7 (m, 2H), 8.30 (t, J = 8.1 Hz, 1H)
FABMS (m/e) 547 $(M+H)^+$ molecular formula $C_{26}H_{25}F_3N_4O_6$ = 546
(2) Substantially the same manner as that in Example 1 (2) was repeated except that 2-(4-allyloxycarbonylamino-3-fluorophenyl)-5-amino-6,8-difluoro-7-(4-methylpiperazinyl)carbonyloxymethyl-4H-1-benzopyran-4-one (294 mg, 0.539 mmol) was used, to give compound 2 (232 mg, yield: 93%).
$^1$H NMR (270 MHz, $CDCl_3$) δ(ppm) 2.31 (s, 3H), 2.39 (brs, 4H), 3.52 (brs, 4H), 4.20 (brs, 2H), 5.32 (s, 2H), 6.22 (brs, 2H), 6.48 (s, 1H), 6.84 (t, J = 8.4 Hz, 1H), 7.5-7.6 (m, 2H)
FABMS (m/e) 463 $(M+H)^+$ molecular formula $C_{22}H_{21}F_3N_4O_4$ = 462

Example 3

5-Amino-2-(4-amino-3-fluorophenyl)-7-[N-(2-dimethylaminoethyl)carbonyloxymethyl]-6,8-difluoro-4H-1-benzopyran-4-one (compound 3)

(1) Substantially the same manner as that in Example 1 (1) was repeated except that Compound (IVa) (420 mg, 1.00 mmol) obtained in Reference Example 3 was used and N,N-dimethylethylenediamine (0.33 mL, 3.0 mmol) was used instead of diethylamine, to give 2-(4-allyloxycarbonylamino-3-fluorophenyl)-5-amino-7-[N-(2-dimethylaminoethyl)carbamoyloxymethyl]-6,8-difluoro-4H-1-benzopyran-4-one (265 mg, yield: 50%).
$^1$H NMR (90 MHz, $CDCl_3$) δ (ppm) 2.23 (s, 6H), 2.42 (t, J = 5.9 Hz, 2H), 3.28 (q, J = 5.9 Hz, 2H), 4.72 (d, J = 5.7 Hz, 2H), 5.1-5.5 (m, 2H), 5.30 (s, 2H), 5.7-6.2 (m, 1H), 6.22 (brs, 2H), 6.56 (s, 1H), 7.05 (brs, 2H), 7.5-7.7 (m, 2H), 8.31 (t, J = 8.1 Hz, 1H)
FABMS (m/e) 535 $(M+H)^+$ molecular formula $C_{25}H_{25}F_3N_4O_6$ =534
(2) Substantially the same manner as that in Example 1 (2) was repeated except that 2-(4-allyloxycarbonylamino-3-fluorophenyl)-5-amino-7-[N-(2-dimethylaminoethyl)carbamoyloxymethyl]-6,8-difluoro-4H-1-benzopyran-4-one (265 mg, 0.496 mmol) was used, to give compound 3 (176 mg, yield: 79%).
$^1$H NMR (270 MHz, $CDCl_3$) δ(ppm) 2.22 (s, 6H), 2.43 (t, J = 5.4 Hz, 2H), 3.29 (q, J = 5.4 Hz, 2H), 4.20 (brs, 2H), 5.29 (s, 2H), 5.38 (m, 1H), 6.21 (brs, 2H), 6.47 (s, 1H), 6.83 (t, J = 8.9 Hz, 1H), 7.5-7.6 (m, 2H)
FABMS (m/e) 451 $(M+H)^+$ molecular formula $C_{21}H_{21}F_3N_4O_4$ =450

Example 4

5-Amino-2-(4-amino-3-fluorophenyl) -7-chloroacetoxymethyl-6,8-difluoro-4H-1-benzopyran-4-one (Compound 4)

Chloroacetic acid (100 g, 1.06 mol) and sulfuric acid (30 mL) were added to Compound (IIa) (10.8 g, 20.0 mmol, 6,8-difluoro-2-(3-fluoro-4-pivaloylaminophenyl)7-hydroxymethyl-5-pivaloylamino-4H-1-benzopyran-4-one) obtained in Reference Example 1, and the mixture was stirred at 100 °C for 20 minutes. The reaction solution was poured into 1 L of ice-water and the mixture was extracted with ethyl acetate. The organic layer was washed with brine and dried over anhydrous sodium sulfate, and the solvent was distilled off under reduced pressure. The residue was triturated with diisopropyl ether, to give compound 4 (8.00 g, yield: 97%).
$^1$H NMR (90 MHz, DMSO-$d_6$) δ (ppm) 4.40 (s, 2H), 5.36 (s, 2H), 6.00 (brs, 2H), 6.66 (s, IH), 6.87 (t, J = 8.9Hz, 1H), 7.06 (brs, 2H), 7.5-7.7 (m, 2H)
FAB-MS (m/e) 413 $(M+H)^+$ molecular- formula $C_{18}H_{12}{}^{35}ClF_3N_20_4$ = 412

Example 5

5-Amino-2-(4-amino-3-fluorophenyl)-7-(3-bromopropanoyl)oxymethyl-6,8-difluoro-4H-1-benzopyran-4-one (Compound 5)

3-Bromopropionic acid (7.65 g, 50.0 mmol) and 1.5mL of sulfuric acid (1.5 mL) were added to Compound (IIa) (504 mg, 1.00 mmol), obtained in Reference Example 1, and the mixture was stirred at 100 °C for 10 minutes. The reaction solution was poured into 100 mL of ice-water and the mixture was extracted with ethyl acetate. The organic layer was washed with brine and dried over anhydrous sodium sulfate, and the solvent was distilled off under reduced pressure. The residue was purified by silica gel column chromatography (chloroform:acetonitrile = 19:1), to give compound 5 (163 mg, yield: 35%).
$^1$H NMR (90 MHz, $CDCl_3$) δ (ppm) 2.97 (t, J = 6.8Hz, 2H), 3.58 (t, J = 6.8Hz, 2H), 5.36(t, J = 1.5Hz, 2H), 6.49 (s, 1H), 6.83 (t, J = 8.7Hz, 1H), 7.4-7.7 (m, 2H)
FAB-MS (m/e) 473, 471 $(M+H)^+$ molecular formula $C_{19}H_{14}{}^{79}BrF_3N_20_4$ = 470

Example 6

5-Amino-2-(4-amino-3-fluorophenyl) -7-dimethylaminoacetoxymethyl-6,8-difluoro-4H-1-benzopyran-4-one (Compound 6)

Compound 4 (1.50 g, 3.63 mmol)obtained in Example 4 was dissolved in dimethylformamide (30 mL). Dimethylamine hydrochloride (1.48 g, 18.2 mmol) and potassium carbonate (2.50 g, 18.2 mmol) were added to the reaction

mixture, and the mixture was stirred at 50 °C for 30 minutes. Water was added to the reaction solution and the mixture was extracted with ethyl acetate. The organic layer was washed with water and brine and dried over anhydrous sodium sulfate, and the solvent was distilled off under reduced pressure. The residue was recrystallized from ethyl acetate/n-hexane, to give compound 6 (1.18 g, yield: 77%). This compound was dissolved in ethyl acetate, then 1 N hydrochloric acid/2-propanol solution (3 mL) was added to the solution. The precipitated crystals were collected by filtration, to give a hydrochloride of compound 6.

$^1$H NMR (270 MHz, DMSO-$d_6$) δ (ppm) 2.84 (s, 6H), 4.27 (s, 2H), 5.42 (s, 2H), 6.73 (s, 1H), 6.87 (t, J = 8.9Hz, 1H), 7.5-7.7 (m, 2H), 10.3 (brs, 1H)

FAB-MS (m/e) 422 $(M+H)^+$ molecular formula $C_{20}H_{18}F_3N_30_4$ = 421

Example 7

5-Amino-2-(4-amino-3-fluorophenyl)-7-(3-dimethylaminopropionyl) oxymethyl-6,8-difluoro-4H-1-benzopyran-4-one (Compound 7)

Compound 5 (130 mg, 0.276 mmol) obtained in Example 5 was dissolved in dimethylformamide (5 mL). Dimethylamine hydrochloride (112 mg, 1.38 mmol) and diisopropylethylamine (0.24 mL, 1.38 mmol) were added to the reaction mixture, and the mixture was stirred at 50 °C for 30 minutes. Water was added to the reaction solution and the mixture was extracted with ethyl acetate. The organic layer was washed, with water and brine and dried over anhydrous sodium sulfate, and the solvent was distilled off under reduced pressure. The residue was purified by silica gel column chromatography (chloroform:methanol = 9:1), to give compound 7 (122 mg, yield: 86%), which was converted to a hydrochloride according to the same manner as that in Example 6.

$^1$H NMR (270 MHz, DMSO-$d_6$) δ (ppm) 2.75 (d, J = 4.5Hz, 6H), 2.93 (t, J = 7.4Hz, 2H), 3.31 (t, J = 7.4Hz, 2H), 5.31 (s, 2H), 6.10 (brs, 2H), 6.72 (s, 1H), 6.88 (t, J = 8.9Hz, 1H), 7.13 (brs, 2H), 7.60 (dd, J = 8.4 Hz, 2.0Hz, 1H), 7.66 (dd, J = 12.9 Hz, 2.0Hz, 1H), 10.2 (brs, 1H)

FAB-MS (m/e) 436 $(M+H)^+$ molecular formula $C_{21}H_{20}F_3N_3O_4$ = 435

Example 8

5-A-mino-2-(4-amino-3-fluorophenyl)-7-(4-dimethylaminobutyryl)oxymethyl-6, 8-difluoro-4H-1-benzopyran-4-one (Compound 8)

(1) 4-Dimethylaminobutyric acid hydrochloride (3.99 g, 23.8 mmol) was dissolved in dimethylformamide (50 mL). N,N'-carbonyldiimidazole (3.86 g, 23.8 mmol) was added to the mixture, and the mixture was stirred at 80 °C for 2.5 hours. Compound (IVa) (1.00 g, 23.8 mmol) obtained in Reference Example 3 was added thereto and the mixture was further stirred at the same temperature for 2 hours. An aqueous saturated solution of sodium bicarbonate was added to the reaction solution and the mixture was extracted with ethyl acetate. The organic layer was washed with brine and dried over anhydrous sodium sulfate, and the solvent was distilled off under reduced pressure. The residue was purified by silica gel column chromatography (chloroform:methanol = 9:1), to give 2-(4-allyloxycarbonylamino-3-fluorophenyl)-5-amino-7-(4-dimethylaminobutyryl)oxymethyl-6,8-difluoro-4H-1-benzopyran-4-one (1.27 g, yield: 100%).

$^1$H NMR (90 MHz, $CDCl_3$) δ (ppm) 1.6-2.0 (m, 2H), 2.1-2.5 (m, 4H), 2.23 (s, 6H), 4.72 (d, J = 5.7Hz, 2H), 5.2-5.5 (m, 2H), 5.30 (s, 2H), 5.7-6.2 (m, 1H), 6.23 (brs, 2H), 6.56 (s, 1H), 7.10 (brs, 1H), 7.5-7.7 (m, 2H), 8.31 (t, J = 7.9Hz, 1H)

FAB-MS (m/e) 534 $(M+H)^+$ molecular formula- $C_{26}H_{26}F_3N_3O_6$ = 533

(2) Substantially the same manner as that in Example 1 (2) was repeated except that the above 2-(4-allyloxycarbonylamino-3-fluorophenyl)-5-amino-7-(4-dimethylaminobutyryl)oxymethyl-6,8-difluoro-4H-1-benzopyran-4-one (1.27 g, 2.38 mmol) was used, to give compound 8 (567 mg, yield: 53%).

$^1$H NMR (270 MHz, DMSO-$d_6$) δ (ppm) 1.94 (q, J = 7.9Hz, 2H), 2.48 (t, J = 7.9Hz, 2H), 2.73 (a, 6H), 3.04 (m, 2H), 5.27 (a, 2H), 6.14 (brs, 2H), 6.72 (s, 1H), 6.87 (t, J = 8.4Hz, 1H), 7.13 (brs, 2H), 7.60 (dd, J=8.4 Hz, 2.0Hz, 1H), 7.66 (dd, J = 12.9 Hz, 2.0Hz, 1H), 10.2 (brs, 1H)

FAB-MS (m/e) 450 $(M+H)^+$ molecular formula $C_{22}H_{22}F_3N_3O_4$ = 449

Example 9

5-Amino-2- (4-amino-3-fluorophenyl)-6,8-difluoro-7-piperidinoacetoxymethyl-4H-1-benzopyran-4-one (Compound 9)

Compound 4 (515 mg, 1.25 mmol) obtained in Example 4 was dissolved in dimethylformamide (10 mL). Piperidine (0.62 mL, 6.2 mmol) and diisopropylethylamine (0.22 mL, 1.25 mmol) were added to the mixture, and the mixture was stirred at 50 °C for 2 hours. Water was added to the reaction solution and the mixture was extracted with ethyl acetate. The organic layer was washed with water and brine and dried over anhydrous sodium sulfate, and the solvent was distilled off under reduced pressure. The residue was purified by silica gel column chromatography (chloroform: acetonitrile = 4:1) and recrystallized from ethyl acetate, to give compound 9 (441 mg, yield: 77%).
$^1$H NMR (270 MHz, $CDCl_3$) δ (ppm) 1.44 (quint., J = 5.4Hz, 2H), 1.64 (quint., J = 5.4Hz, 4H), 2.57 (t, J = 5.4Hz, 2H), 3.28 (s, 2H), 4.21 (brs, 2H), 5.34 (s, 2H), 6.23 (brs, 2H), 6.49 (s, 1H), 6.84 (t, J = 8.9Hz, 1H), 7.5-7.6 (m, 2H)
FAB-MS (M/Z) 462 $(M+H)^+$ molecular formula $C_{23}H_{22}F_3N_3O_4$ = 461

Example 10

5-Amino-2-(4-amino-3-fluorophenyl)-6,8-difluoro-7-(4-methylpiperazinyl)acetoxymethyl-4H-1-benzopyran-4-one (Compound 10)

Substantially the same manner as that in Example 9 was repeated except that methylpiperazine (0.69 mL, 6.2 mmol) was used instead of piperidine, to give compound 10 (408 mg, yield: 69%).
$^1$H NMR (270 MHz, $CDCl_3$) δ (ppm) 2.33 (s, 3H), 2.55 (brs, 4H), 2.66 (brs, 4H), 3.28 (s, 2H), 4.21 (brs, 2H), 5.34 (s, 2H), 6.23 (brs, 2H), 6.49 (s, 1H), 6.84 (t, J = 8.4Hz, 1H), 7.5-7.6 (m, 2H)
FAB-MS (m/e) 477 $(M+H)^+$ molecular formula $C_{23}H_{23}F_3N_3O_4$ = 476

Example 11

5-Amino-2-(4-amino-3-fluorophenyl)-6,8-difluoro-7-morpholinoacetoxymethyl-4H-1-benzopyran-4-one (Compound 11)

Substantially the same manner as that in Example 9 was repeated except that morpholine (0.55 mL, 6.2 mmol) was used instead of piperidine, to give compound 11 (350 mg, yield: 61%).
$^1$H NMR (270 MHz, DMSO-$d_6$) δ (ppm) 2.4-2.6 (m, 4H), 3.56 (t, J = 4.7Hz, 4H), 5.28 (s, 2H), 6.10 (brs, 2H), 6.71 (s, 1H), 6.87 (t, J = 8.9Hz, 1H), 7.12 (brs, 2H), 7.60 (dd, J = 8.4 Hz, 2.0Hz, 1H), 7.66 (dd, J = 12.9 Hz, 2.0Hz, 1H)
FAB-MS (m/e) 464 $(M+H)^+$ molecular formula $C_{22}H_{20}F_3N_3O_5$ = 463

Example 12

5-Amino-2-(4-amino-3-fluorophenyl) -6, 8-difluoro-7-(4-hydroxypiperidino)acetoxymethyl-4H-1-benzopyran-4-one (Compound 12)

Substantially the same manner as that in Example 9 was repeated except that 4-hydroxypiperidine (633 mg, 6.2 mmol) was used instead of piperidine, to give compound 12 (518 mg, yield: 89%).
$^1$H NMR (270 MHz, DMSO-$d_6$) δ (ppm) 1.3-1.5 (m, 2H), 1.6-1.8 (m, 2H), 2.1-2.3 (m, 2H), 2.6-2.8 (m, 2H), 3.23 (s, 2H), 3.3-3.5 (m, 1H), 4.53 (d, J = 4.0 Hz, 1H), 5.26 (brs, 2H), 6.12 (brs, 2H), 6.71 (s, 1H), 6.87 (t, J = 8.9Hz, 1H), 7.12(brs, 2H), 7.60 (dd, J = 8.4 Hz, 2.0Hz, 1H), 7.67 (dd, J = 12.9 Hz, 2.0Hz, 1H)
FAB-MS (m/e) 478 $(M+H)^+$ molecular formula $C_{23}H_{22}F_3N_3O_5$ = 477

Example 13

5-Amino-2-(4-amino-3-fluorophenyl)-7-[N-(2-dimethylaminoethyl)-N-methylamino)acetoxymethyl-6,8-difluoro-4H-1-benzopyran-4-one (Compound 13)

Substantially the same manner as that in Example 9 was repeated except that N,N,N'-trimethylethylenediamine (0.80 mL, 6.2 mmol) was used instead of piperidine, to give compound 13 (398 mg, yield: 67%).
$^1$H NMR (270 MHz, $CDCl_3$) δ (ppm) 2.33 (s, 6H), 2.43 (s, 3H), 2.51 (t, J = 6.4Hz, 2H), 2.74 (t, J = 6.4Hz, 2H), 3.41 (s, 2H), 4.20 (brs, 2H), 5.33 (s, 2H), 6.24 (brs, 1H), 6.49 (s, 1H), 6.85 (t, J = 8.7Hz, 1H), 7.5-7.6 (m, 2H)
FAB-MS (m/e) 479 $(M+H)^+$ molecular formula $C_{23}H_{25}F_3N_4O_4$ = 478

Example 14

5-Amino-2-(4-amino-3-fluorophenyl)-6,8-difluoro-7-methoxycarbonylmethylaminoacetoxymethyl-4H-1-benzopyran-4-one (Compound 14)

Substantially the same manner as that in Example 9 was repeated except that glycine methyl ester hydrochloride (783 mg, 6.2 mmol) was used instead of piperidine, to give compound 14 (291 mg, yield: 50%).
$^1$H NMR (270 MHz, $CDCl_3$) δ (ppm) 3.50 (s, 2H), 3.53 (s, 2H), 3.73 (s, 3H), 4.20 (brs, 2H), 5.36 (s, 2H), 5.41 (brs, 1H), 6.24 (brs, 2H), 6.49 (s, 1H), 6.84 (t, J = 8.9Hz, 1H), 7.5-7.6 (m, 2H) FAB-MS (M/Z) 466 $(M+H)^+$ molecular formula $C_{21}H_{18}F_3N_3O_6$ = 465

Example 15

7-Allyloxycarbonyloxymethyl-5-amino-2-(4-amino-3-fluorophenyl)-6,8-difluoro-4H-1-benzopyran-4-one (Compound 15)

Compound (IIIa) (336 mg, 1.00ml) obtained in Reference Example 2 was dissolved in pyridine (20 mL). Diallyl pyrocarbonate (1.0 mL, 5.0 mmol) and 4-dimethylaminopyridine (27 mg, 0.20 mmol) were added to the mixture under ice-cooling, and the mixture was stirred at room temperature for 4 hours. Water was added to the reaction solution and the mixture was extracted with ethyl acetate. The organic layer was washed twice with 1 N hydrochloric acid and each once with water and brine, and dried over anhydrous sodium sulfate, and the solvent was distilled off under reduced pressure. The residue was purified by silica gel column chromatography (chloroform:methanol = 200:1) and recrystallized from ethyl acetate/n-hexane, to give compound 15 (172 mg, yield: 41%).
$^1$H NMR (270 MHz, $CDCl_3$) δ (ppm) 4.67 (d, J = 5.9Hz, 2H), 5.28 (dd, J = 10.4 Hz, 1.0Hz, 1H), 5.37 (dd, J=17.3Hz, 1.0 Hz, 1H), 5.38 (s, 2H), 5.96 (ddd, J = 17.3 Hz, 10.4 Hz, 5.9Hz, 1H), 6.49 (s, 1H), 6.84 (t, J = 8.9Hz, 1H), 7.5-7.6 (m, 2H) FAB-MS (m/e) 421 $(M+H)^+$ molecular formula $C_{20}H_{15}F_3N_2O_5$ = 420

Example 16

5-Amino-2-(4-amino-3,5-dichlorophenyl)-7-chloroacetoxymethyl-6,8-difluoro-4H-1-benzopyran-4-one (Compound 16)

Chloroacetic acid (26.7 g, 283 mmol) and sulfuric acid (8.0 mL) were added to Compound (IIb) (3.14 g, 5.66 mmol) obtained in Reference Example 5, and the mixture was stirred at 100 °C for 20 minutes. The reaction solution was cooled at room temperature and poured into ice-water, and the precipitated crystals were collected by filtration, to give compound 16 (2.40 g, yield: 91%).
$^1$H NMR (90 MHz, DMSO-$d_6$) δ (ppm) 4.44 (s, 2H), 5.36 (t, J = 1.5Hz, 2H), 6.84 (s, 1H), 7.88 (s, 2H)
FAB-MS (m/e) 463 $(M+H)^+$ molecular formula $C_{18}H_{11}{}^{35}Cl_3F_2N_2O_4$ = 462

Example 17

5-Amino-2-(4-amino-3,5-dichlorophenyl)-7-dimethylaminoacetoxymethyl-6,8-difluoro-4H-1-benzopyran-4-one (Compound 17)

Compound 16 (2.00 g, 4.31 mmol) obtained in Example 16 was dissolved in dimethylformamide (80 mL). Dimethylamine hydrochloride (1.76 g, 21.6 mmol) and diisopropylethylamine (3.75 mL, 21.6 mmol) were added to the mixture, and the mixture was stirred at 50 °C for 3 hours. Water was added to the reaction solution and the precipitated crystals were collected by filtration. The crystals were purified by silica gel column chromatography (chloroform:methanol = 9:1), to give compound 17 (1.68 g, yield: 83%).
$^1$H NMR (270 MHz, DMSO-$d_6$) δ (ppm) 2.25 (s, 6H), 3.24 (s, 2H), 5.27 (brs, 2H), 6.37 (brs, 2H), 6.83 (s, 1H), 7.11 (brs, 2H), 7.87 (s, 2H)
FAB-MS (m/e) 472 $(M+H)^+$ molecular formula $C_{20}H_{17}{}^{35}Cl_2F_2N_3O_4$ = 471

Reference Example 1

6,8-Difluoro-2-(3-fluoro-4-pivaloylaminophenyl)-7-hydroxymethyl-5-pivaloylamino-4H-1-benzopyran-4-one (Compound IIa)

2,4-Difluorophenol (104 g, 796 mmol) was dissolved in dichloromethane (800 mL). Triethylamine (132 mL)

and ethyl chloroformate (92.0 mL) were added to the mixture under ice-cooling and the mixture was stirred at -10 to 0 °C for 2 hours. The reaction mixture was washed with brine and dried over anhydrous magnesium sulfate. The solvent was distilled off under reduced pressure to give 1-ethoxycarbonyloxy-2,4-difluorobenzene (156 g, yield: 97%).
$^1$H NMR (90 MHz, $CDCl_3$) δ (ppm) 1.39 (t, J = 7.0Hz, 3H), 4.33 (q, J = 7.0Hz, 2H), 6.7-7.3 (m, 3H)
EI-MS (m/e) 202 $M^+$ molecular formula $C_9H_8F_2O_3$ = 202

1-Ethoxycarbonyloxy-2,4-difluorobenzene (50.5 g, 250 mmol) obtained above was dissolved in conc. sulfuric acid (115 mL), and fuming nitric acid (15.9 mL) was added to the mixture. While adding the acid, the temperature of the mixture was kept at 10 to 20 °C and the reaction mixture was stirred at the same temperature for 1 hour. The reaction mixture was poured into ice-water and the mixture was extracted with ethyl acetate (500 mL). The organic layer was washed with brine twice and dried over anhydrous sodium sulfate. After the solvent was distilled off under reduced pressure, the residue was dissolved in methanol (1.0 L), and water (50 mL) and sodium bicarbonate (40 g) was added to the mixture. The reaction mixture was stirred at room temperature for 16 hours. The reaction mixture was filtrated and methanol was distilled off under reduced pressure. Water (200 mL) was added, and the pH of the solution was adjusted to 5. The mixture was extracted with ethyl acetate (200 mL) twice. The organic layer was washed once with water (400 mL) and brine (400 mL), dried over anhydrous sodium sulfate. The solvent was distilled off under reduced pressure to give 2,4-difluoro-5-nitrophencl (41.6 g, yield: 95%).
$^1$H NMR (90 MHz, $CDCl_3$) δ (ppm) 7.23 (t, J = 9.9 Hz, 1H), 7.76 (dd, J = 8.6 Hz, 7.3Hz, 1H)
EI-MS (m/e) 175 $M^+$ molecular formula $C_6H_3F_2NO_3$ = 175

2,4-Difluoro-5-nitrophenol (24.9 g, 142 mmol) obtained above was dissolved in ethyl acetate (150 mL) and 10% palladium on activated carbon (5.0 g) was added to the mixture. The reaction mixture was stirred under hydrogen stream at 50 to 60 °C for 27 hours. After the atmosphere in reactor was replaced with nitrogen, the reaction mixture was filtrated with suction. The solvent in filtrate was distilled off under reduced pressure, the residue was triturated with hexane to give 5-amino-2,4-difluorophenol (19.8 g, yield: 96%).
$^1$H NMR (90 MHz, $CDCl_3$) δ (ppm) 4.75 (brs, 2H), 6.37 (t, J = 9.1 Hz, 1H), 6.87 (t, J = 11.1 Hz, 1H), 9.21 (s, 1H)
EI-MS (m/e) 145 $M^+$ molecular formula $C_6H_5F_2NO$ = 145

5-Amino-2,4-difluorophenol (18.9 g, 130 mmol) obtained above was dissolved in pyridine (45 mL) and pivaloyl chloride (16.0 mL) was added dropwise to the mixture for 8 minutes under ice-cooling. The reaction mixture was stirred for additional 30 minutes at the same temperature. Hydrochloric acid (1 N) was added to the reaction mixture and the mixture was extracted with ether. The organic layer was washed with 1 N hydrochloric acid, water, and brine once respectively, and dried over anhydrous magnesium sulfate. The solvent was distilled off under reduced pressure and the residue was triturated with hexane to give 2,4-difluoro-5-pivaloylaminophenol (27.0 g, yield: 91%).
$^1$H NMR (90 MHz, $CDCl_3$) δ (ppm) 1.35 (s, 9H), 6.90 (t, J = 10.4Hz, 1H), 7.65 (brs, 1H), 7.94 (brs, 1H), 8.24 (dd, J = 9.1 Hz, 8.0Hz, 1H)
EI-MS (m/e) 229 $M^+$ molecular formula $C_{11}H_{13}F_2NO_2$ = 229

2,4-Difluoro-5-pivaloylaminophenol (2.15 g, 9.39 mmol) was dissolved in dichloromethane (40 mL), and 3,4-dihydro-2H-pyran (4.3 mL) and camphorsulfonic acid (44 mg) were added to the mixture. The reaction mixture was stirred for 4.3 hours at room temperature. The reaction mixture was added to 5% aqueous potassium carbonate and the mixture was extracted with chloroform. The organic layer was washed with water and brine once respectively and dried over anhydrous magnesium sulfate. The solvent was distilled off under reduced pressure and the residue was triturated with hexane to give 2-(2,4-difluoro-5-pivaloylaminophenoxy)tetrahydropyran (2.51 g, yield: 85%).
$^1$H NMR (90MHz, $CDCl_3$) δ (ppm) 1.31 (s, 9H), 1.4-2.2 (m, 6H), 3.4-4.2 (m, 2H), 5.43 (brs, 1H), 6.89 (t, J = 10.4Hz, 1H), 7.44 (brs, 1H), 8.25 (t, J = 8.5Hz, 1H)
EI-MS (m/e) 313 $M^+$ molecular formula $C_{15}H_{21}F_2NO_3$ = 313

Under the atmosphere of argon, diisopropylamine (35 mL, 250 mmol) was dissolved in tetrahydrofuran (120 mL), n-butyl lithium (1.6 M solution in hexane, 140 mL, 224 mmol) was added dropwise to the reaction mixture under ice-cooling, while n-butyl lithium solution was added, and the temperature of the mixture was kept at 0 to 5 °C. Then the reaction mixture was cooled to under -60 °C. The solution of 2-(2,4-difluoro-5-pivaloylaminophenoxy) tetrahydropyran (31.3 g, 100 mmol) obtained above in tetrahydrofuran (200 mL) was added dropwise to the reaction mixture at the internal temperature of under -60 °C for 1 hour. The reaction mixture was stirred at the same temperature for additional 20 minutes. Dimethylformamide (15.5 mL, 200 mmol) was added dropwise to the mixture, keeping the temperature of the mixture at under -60 °C. The reaction mixture was stirred for additional 30 minutes at room temperature. Water was added to the mixture and the mixture was extracted with ethyl acetate twice. The organic layer was washed with water and brine once respectively and dried over anhydrous sodium sulfate. The solvent was distilled off under reduced pressure. The residue was recrystallized from ethyl acetate/hexane (140 mL/100 mL) to give 2,6-difluoro-3-pivaloylamino-5-(2-tetrahydropyranyloxy)benzaldehyde (19.0 g, yield: 56%). The mother liquor was concentrated and the residue was purified by silica gel column chromatography (ethyl acetate:hexane = 5:1 to 4:1) to give the additional Compound (6.94g, yield: 20%).
$^1$H NMR (90 MHz, $CDCl_3$) δ (ppm) 1.32 (s, 9H), 1.5-2.0 (m, 6H), 3.5-4.1 (m, 2H), 5.47 (brs, 1H), 7.49 (brs, 1H), 8.55

(t, J= 8.4Hz, 1H), 10.4 (s, 1H)
FAB-MS (m/e) 342 $(M+H)^+$ molecular formula $C_{17}H_{21}F_2NO_4$ = 341

2,6-Difluoro-3-pivaloylamino-5-(2-tetrahydropyranyloxy)benzaldehyde (25.4 g, 74.5 mmol) obtained above was dissolved in methanol (300 mL). Under ice-cooling, sodium borohydride (1.41 g, 37.3 mmol) was added to the mixture and the reaction mixture was stirred for 15 minutes at the same temperature. Water was added to the mixture and the mixture was concentrated to ca. 100 mL. The mixture was extracted with ethyl acetate twice and the organic layer was washed with brine once and dried over anhydrous sodium sulfate. The solvent was distilled off under reduced pressure to give 2,6-difluoro-3-pivaloylamino-5-(2-tetrahydropyranyloxy)phenylmethanol (25.5 g, yield: 100%).
$^1H$ NMR (90 MHz, $CDCl_3$) δ (ppm) 1.31 (s, 9H), 1.5-2.1 (m, 6H), 3.5-4.1 (m, 2H), 4.78 (brs, 2H), 5.43 (brs, 1H), 7.49 (brs, 1H), 8.20 (t, J = 8.6Hz, 1H)
FAB-MS (m/e) 344 $(M+H)^+$ molecular formula $C_{17}H_{23}F_2NO_4$ = 343

2, 6-Difluoro-3-pivaloylamino-5- (2-tetrahydropyranyloxy)phenylmethanol (74.9 g, 218 mmol) was dissolved in dichloromethane (700 mL) and 3,4-dihydro-2H-pyran (40 mL, 436 mmol) and camphorsulfonic acid (1.00 g, 4.36 mmol) were added to the mixture. The reaction mixture was stirred for 30 minutes at room temperature. The reaction mixture was poured into aqueous solution of sodium hydroxide (0.5 N, 500 mL) and the mixture was extracted with chloroform. The organic layer was washed with brine and dried over anhydrous sodium sulfate. The solvent was distilled off under reduced pressure to give 2-[2,6-difluoro-5-pivaloylamino-3-(2-tetrahydropyranyloxy)phenylmethoxy]tetrahydropyran (92.1 g, yield: 99%).
$^1H$ NMR (90 MHz, $CDCl_3$) δ (ppm) 1.31 (s, 9H), 1.4-2.0 (m, 12H), 3.4-4.1 (m, 4H), 4.4-5.0 (m, 3H), 5.43 (brs, 1H), 7.46 (brs, 1H), 8.24 (t, J = 8.4Hz, 1H)
FAB-MS (m/e) 428 $(M+H)^+$ molecular formula $C_{22}H_{31}F_2NO_5$ = 427

Under the stream of nitrogen, 2-[2,6-Difluoro-5-pivaloylamino-3-(2-tetrahydropyranyloxy)phenylmethoxy]-tetrahydropyran (92.1 g, 216 mmol) was dissolved in tetrahydrofuran (800 mL) and the mixture was cooled until the temperature of the mixture became under -60 °C. n-Butyl lithium (1.6 M solution in hexane, 340 mL, 545 mmol) was added dropwise thereto while the temperature of the mixture was kept at -60 °C, and then the temperature was adjusted to -30 °C. The mixture was stirred at the same temperature for 5 minutes, and then cooled again until the temperature of the mixture became under -60 °C. Ethyl chloroformate (42.0 mL, 440 mmol) was added dropwise, while the temperature of the mixture was kept under -60 °C. Then the mixture was stirred for 10 minutes. Water was added to the reaction mixture and the mixture was heated to room temperature and extracted with ethyl acetate. The organic layer was washed with water and brine and dried over anhydrous sodium sulfate. The solvent was distilled off under reduced pressure and the residue was purified by silica gel column chromatography (hexane:ethyl acetate = 5:1 to 3:1) to give ethyl 3,5-difluoro-2-pivaloylamino-6-(2-tetrahydropyranyloxy)-4-[(2-tetrahydropyranyl)oxymethyl]benzoate (86.7 g, yield: 80%).
$^1H$ NMR (90 MHz, $CDCl_3$) δ (ppm) 1.29 (s, 9H), 1.38 (t, J = 7.0Hz, 3H), 1.4-2.1 (m, 12H), 3.4-4.1 (m, 4H), 4.35 (q, J = 7.0Hz, 2H), 4.5-4.9 (m, 3H), 5.32 (brs, 1H), 7.57 (brs, 1H)
FAB-MS (m/e) 500 $(M+H)^+$ molecular formula $C_{25}H_{35}F_2NO_7$ = 499

Under the atmosphere of argon, sodium hydride (60% in oil, 7.88 g, 197 mmol) was washed with hexane 3 times, suspended in mixed solvent of 1,4-dioxane (60 mL) and toluene (60 mL), and refluxed under heating. The solution of ethyl 3,5-difluoro-2-pivaloylamino-6-(2-tetrahydropyranyloxy)-4-[(2-tetrahydropyranyl)oxymethyl]benzoate (30.9 g, 61.9 mmol) obtained above and 3'-fluoro-4'pivaloylaminoacetophenone (13.3 g, 56.3 mmol) described below in Reference Example 4 in the mixed solvent of 1,4-dioxane (150 mL) and toluene (150 mL) was added dropwise to the reaction mixture for 10 minutes and the mixture was refluxed under heating for 4 hours. Then the mixture was cooled on ice bath. Water was added to the reaction mixture and the mixture was extracted with ethyl acetate 3 times. The organic layer was washed with brine and dried over anhydrous sodium sulfate. The solvent was distilled off under reduced pressure to give crude 1-{3,5-difluoro-2-pivaloylamino-6-(2-tetrahydropyranyloxy)-4-[(2-tetrahydropyranyl) oxymethyl]phenyl}-3-(3-fluoro-4-pivaloylaminophenyl)-1,3-propanedione. This crude product was used in the next step without further purification.

The above crude compound (41. 0 g) was dissolved in ethanol (240 mL) and hydrochloric acid (60 mL) was added to the mixture and the mixture was stirred at room temperature overnight. Water was added to the mixture and precipitated crystals were separated by filtration. The crystals were triturated with mixed solvent of ethyl acetate (200 mL) and diisopropylether (300 mL) to give Compound (IIa) (13.8 g, yield: 49%). The mother liquor was concentrated and the residue was recrystallized from chloroform/methanol to give additional Compound (IIa)(3.66 g, yield: 13%). The mother liquor was concentrated and the residue was purified by silica gel column chromatography (chloroform: acetonitorile = 9:1) to give additional Compound (IIa)(1.60 g, yield: 5.6%).
$^1H$ NMR (90 MHz, DMSO-$d_6$) δ (ppm) 1.27 (s, 9H), 1.29 (s, 9H), 4.6-4.7 (m, 2H), 5.56 (t, J = 5.7Hz, 1H), 7.07 (s, 1H), 7.7-8.0 (m, 3H), 9.20 (brs, 1H), 10.0 (brs, 1H)
EI-MS (m/e) 504 $M^+$ molecular formula $C_{26}H_{27}F_3N_2O_5$ = 504

Reference Example 2

5-Amino-2-(4-amino-3-fluorophenyl)-6,8-difluoro-7-hydroxymethyl-4H-1-benzopyran-4-one (Compound (IIIa))

Ethanol (40 mL) and conc. hydrochloric acid (20 mL) were added to the Compound (IIa)(903 mg, 1.79 mmol) obtained above Reference Example 1 and the mixture was refluxed under heating for 6.5 hours. After the reaction mixture was cooled on ice, pH of the solution was adjusted to 7 to 8 by adding aqueous 10 N sodium hydroxide solution. The precipitated crystals were separated by filtration. The crystals were purified by silica gel column chromatography (chloroform:methanol = 40:1 to 9:1) and by trituration with ethyl acetate to give Compound (IIIa)(364 mg, yield: 60%).
$^1$H NMR (270 MHz, DMSO-$d_6$) δ (ppm) 4.5-4.6 (m, 2H), 5.44 (t, J = 4.9Hz, 1H), 6.11 (brs, 2H), 6.69 (s, 1H), 6.87 (t, J = 8.4Hz, 1H), 7.03 (brs, 2H), 7.5-7.7 (m, 2H)
EI-MS (m/e) 336 $M^+$ molecular formula $C_{16}H_{11}F_3N_2O_3$ = 336

Reference Example 3

2- (4-Allyloxycarbonylamino-3-fluorophenyl)-5-amino-6,8-difluoro-7-hydroxymethyl-4H-1-benzopyran-4-one

(Compound IVa)

Compound (IIIa) (6.72 g, 20.0 mmol) claimed in Reference Example 2 was dissolved in pyridine (200 mL) and allyl chloroformate (10.6 mL, 100 mmol) was added dropwise thereto under ice cooling. The reaction mixture was stirred at room temperature for 1.5 hours. Water was added to the mixture and precipitated crystals were separated by filtration. The crystals were dissolved in ethanol (500 mL) and aqueous 2 N sodium hydroxide solution (18 mL) was added to the mixture and the mixture was stirred at room temperature for 1.5 hours. Water was added to the mixture and precipitated crystals were separated by filtration to give Compound (IVa)(6.92 g, yield: 82%).
$^1$H NMR (90 MHz, DMSO-$d_6$) δ (ppm) 4.5-4.7 (m, 4H), 5.1-5.5 (m, 2H), 5.7-6.2 (m, 1H), 6.90 (s, 1H), 7.01 (brs, 2H), 7.7-8.0 (m, 4H), 9.79 (brs, 1H)
FAB-MS (m/e) 421 $(M+H)^+$ molecular formula $C_{20}H_{15}F_3N_2O_5$ = 420

Reference Example 4

3'-Fluoro-4'-pivaloylaminoacetophenone

4-Bromo-2-fluoroaniline (250 g, 1.32 mol) was dissolved in pyridine (500 mL). Pivaloyl chloride (178 mL, 1.45 mol) was added dropwise to the solution and the mixture was stirred for 10 minutes under ice cooling. The reaction mixture was poured into ice-water (1.5 L) and precipitated crystals were separated by filtration. The crystals were washed with aqueous 1 N hydrochloric acid and water and dried under reduced pressure to give 4-bromo-2-fluoro-N-pivaloylaniline (350 g, yield: 97%).

Under the atmosphere of argon, 4-bromo-2-fluoro-N-pivaloylaniline (70.6 g, 258 mmol) obtained above was dissolved in toluene (500 mL). (1-Ethoxyvinyl) tributyltin (108 mL, 310 mmol) and bis (triphenylphosphin)palladium chloride (II) (1.80 g, 2.57 mmol) was added to the solution and the mixture was stirred at 100 °C for 5 hours. The reaction mixture was cooled on ice and 2 N hydrochloric acid (500 mL) was added to the mixture, which was stirred at room temperature for 2 hours. Insoluble materials were separated by filtration. The filtrate was extracted with ethyl acetate once, and an aqueous 10 % ammonium fluoride solution (500 mL) was added to the organic layer. The mixture was stirred at room temperature for 3 hours. Insoluble materials were separated by filtration and the organic layer was washed with water and brine once and dried over anhydrous sodium sulfate. The solvent was distilled off under reduced pressure, and the residue was purified by silica gel column chromatography (hexan:ethyl acetate = 6:1 to 4:1) to give 3'-fluoro-4'-pivaloylaminoacetophenone (60.8 g, yield: 99%).
$^1$H NMR (90 MHz, $CDCl_3$) δ (ppm) 1.35 (s, 9H), 2.57 (s, 3H), 7.6-7.9 (m, 3H), 8.53 (t, J=8.4 Hz, 1H)
EI-MS (m/e) 237 $M^+$ molecular formula $C_{13}H_{16}FNO_2$ = 237

Reference Example 5

2-(3,5-Dichloro-4-pivaloylaminophenyl)-6,8-difluoro-7-hydroxymethyl-5-pivaloylamino-4H-1-benzopyran-4-one (Compound IIb)

Substantially the same manner as that in Reference Example 1 was repeated except that 3',5'-dichloro-4'-pivaloylaminoacetophenone obtained in Reference Example 6 was used instead of 3'-fluoro-4'-pivalcylaminoacetophnone, to give Compound (IIb)(3.34 g, overall yield: 64%).
$^1$H NMR (90 MHz, DMSO-$d_6$) δ (ppm) 1.29 (s, 18H), 4.68 (brs, 2H), 7.22 (s, 1H), 8.16 (s, 2H), 9.51 (brs, 1H), 9.88 (brs, 1H)
FAB-MS (m/e) 555 $(M+H)^+$ molecular formula $C_{26}H_{26}{}^{35}Cl_2F_2N_2O_5$ = 554

Reference Example 6

3'5'-Dichloro-4'-pivaloylaminoacetophenone

Substantially the same manner as that in Reference Example 4 was repeated except that 4-bromo-2,6-dichloroaniline was used instead of 4-bromo-2-fluoroaniline, to give compound 3'5'-dichloro-4'-pivaloylaminoacetophenone (8.93 g, overall yield: 86%).
$^1$H NMR (90 MHz, $CDCl_3$) δ (ppm) 1.38 (s, 9H), 2.58 (s, 3H), 7.26 (brs, 1H), 7.90 (s, 2H)
EI-MS (m/e) 287 $M^+$ molecular formula $C_{13}H_{15}{}^{35}Cl_2NO_2$ = 287

Industrial Availability

The present invention can provide 5-aminoflavone derivatives possessing antibacterial activity, anti-estrogenic activity, and antitumor activity.

## Claims

**1.** A 5-aminoflavone derivative represented by the formula (I):

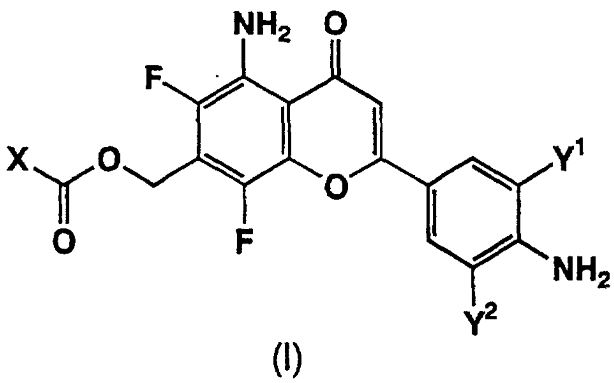

(I)

wherein X represents (i) $C_1$-$C_6$ alkyl substituted with 1 to 3 substituents selected from a group consisting of $NR^3R^4$ {wherein $R^3$ and $R^4$ are the same or different and represent hydrogen, $C_1$-$C_6$ alkyl which may be substituted with $NR^5R^6$ (wherein $R^5$ and $R^6$ are the same or different and represent hydrogen, or $C_1$-$C_6$ alkyl), or $R^3$ and $R^4$ are taken together to form a heterocyclic group containing the sandwiched nitrogen atom in the ring which may be substituted with 1 to 3 substituents selected from a group consisting of $C_1$-$C_6$ alkyl and $NR^7R^8$ (wherein $R^7$ and $R^8$ are the same or different and represent hydrogen, or $C_1$-$C_6$ alkyl)}, halogen, hydroxy, $C_1$-$C_6$ alkoxy, $C_1$-$C_6$ alkoxycarbonyl, $C_1$-$C_6$ alkanoyl, $C_1$-$C_6$ alkanoyloxy, and vinyl,
(ii) $C_1$-$C_6$ alkoxy which may be substituted with 1 to 3 substituents selected from a group consisting of $NR^3R^4$ {wherein $R^3$ and $R^4$ are the same or different and represent hydrogen, $C_1$-$C_6$ alkyl which may be substituted with $NR^5R^6$ (wherein $R^5$ and $R^6$ are the same or different and represent hydrogen, or $C_1$-$C_6$ alkyl), or $R^3$ and $R^4$ are taken together to form a heterocyclic group containing the sandwiched nitrogen atom in the ring which may be substituted with 1 to 3 substituents selected from a group consisting of $C_1$-$C_6$ alkyl and $NR^7R^8$ (wherein $R^7$ and $R^8$ are the same or different and represent hydrogen, or $C_1$-$C_6$ alkyl)}, halogen, hydroxy, $C_1$-$C_6$ alkoxy, $C_1$-$C_6$ alkoxycarbonyl, $C_1$-$C_6$ alkanoyl, $C_1$-$C_6$ alkanoyloxy, and vinyl, or
(iii) $NR^1R^2$ <wherein $R^1$ and $R^2$ are the same or different and represent hydrogen, $C_1$-$C_6$ alkyl which may be substituted with 1 to 3 substituents selected from a group consisting of $NR^3R^4$ {wherein $R^3$ and $R^4$ are the same or different and represent hydrogen, $C_1$-$C_6$ alkyl which may be substituted with $NR^5R^6$ (wherein $R^5$ and $R^6$ are the same or different and represent hydrogen, or $C_1$-$C_6$ alkyl), or $R^3$ and $R^4$ are taken together to form a heterocyclic

group containing the sandwiched nitrogen atom in the ring which may be substituted with 1 to 3 substituents selected from a group consisting of $C_1$-$C_6$ alkyl and $NR^7R^8$ (wherein $R^7$ and $R^8$ are the same or different and represent hydrogen, or $C_1$-$C_6$ alkyl)}, halogen, hydroxy, $C_1$-$C_6$ alkoxy, $C_1$-$C_6$ alkoxycarbonyl, $C_1$-$C_6$ alkanoyl, $C_1$-$C_6$ alkanoyloxy, and vinyl, or $R^1$ and $R^2$ are taken together to form a heterocyclic group containing the sandwiched nitrogen atom in the ring which may be substituted with 1 to 3 substituents selected from a group consisting of $C_1$-$C_6$ alkyl and $NR^7R^8$ (wherein $R^7$ and $R^8$ are the same or different and represent hydrogen, or $C_1$-$C_6$ alkyl)>, and $Y^1$ and $Y^2$ are the same or different and represent hydrogen, halogen, or $C_1$-$C_6$ alkyl, or
a pharmaceutically acceptable salt thereof.

**2.** The 5-aminoflavone derivative according to claim 1, wherein X represents $C_1$-$C_6$ alkyl substituted with 1 to 3 substituents selected from a group consisting of $NR^3R^4$ {wherein $R^3$ and $R^4$ are the same or different and represent hydrogen, $C_1$-$C_6$ alkyl which may be substituted with $NR^5R^6$ (wherein $R^5$ and $R^6$ are the same or different and represent hydrogen, or $C_1$-$C_6$ alkyl), or $R^3$ and $R^4$ are taken together to form a heterocyclic group containing the sandwiched nitrogen atom in the ring which may be substituted with 1 to 3 substituents selected from a group consisting of $C_1$-$C_6$ alkyl and $NR^7R^8$ (wherein $R^7$ and $R^8$ are the same or different and represent hydrogen, or $C_1$-$C_6$ alkyl)}, halogen, hydroxy, $C_1$-$C_6$ alkoxy, $C_1$-$C_6$ alkoxycarbonyl, $C_1$-$C_6$ alkanoyl, $C_1$-$C_6$ alkanoyloxy, and vinyl, or a pharmaceutically acceptable salt thereof.

**3.** The 5-aminoflavone derivative according to claim 1 or 2, wherein at least one of $Y^1$ or $Y^2$ represents halogen, or a pharmaceutically acceptable salt thereof.

**Patentansprüche**

**1.** 5-Aminoflavon-Derivat der Formel (I):

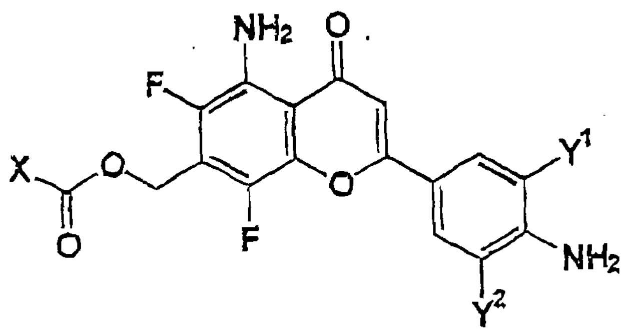

(I)

wobei X

(i) einen $C_1$-$C_6$-Alkylrest darstellt, der mit 1 bis 3 Substituenten substituiert ist, ausgewählt aus einem Rest der Formel $NR^3R^4$ {wobei $R^3$ und $R^4$ gleich oder verschieden sind und ein Wasserstoffatom, einen $C_1$-$C_6$-Alkylrest, der mit einem Rest der Formel $NR^5R^6$ substituiert sein kann (wobei $R^5$ und $R^6$ gleich oder verschieden sind und ein Wasserstoffatom oder einen $C_1$-$C_6$-Alkylrest bedeuten), bedeuten, oder $R^3$ und $R^4$ zusammen genommen einen heterocyclischen Rest bilden, welcher das Stickstoffatom in dem Ring in Sandwich-Stellung enthält, der mit 1 bis 3 Substituenten substituiert sein kann, ausgewählt aus einem $C_1$-$C_6$-Alkylrest und einem Rest der Formel $NR^7R^8$ (wobei $R^7$ und $R^6$ gleich oder verschieden sind und ein Wasserstoffatom oder einen $C_1$-$C_6$-Alkylrest bedeuten)}, einem Halogenatom, einer Hydroxygruppe, einem $C_1$-$C_6$-Alkoxy-, $C_1$-$C_6$-Alkoxycarbonyl-, $C_1$-$C_6$-Alkanoyl-, $C_1$-$C_6$-Alkanoyloxyrest und einer Vinylgruppe,
(ii) einen $C_1$-$C_6$-Alkoxyrest, der mit 1 bis 3 Substituenten substituiert sein kann, ausgewählt aus einem Rest der Formel $NR^3R^4$ {wobei $R^3$ und $R^4$ gleich oder verschieden sind und ein Wasserstoffatom, einen $C_1$-$C_6$-Alkylrest, der mit einem Rest der Formel $NR^5R^6$ substituiert sein kann (wobei $R^5$ und $R^6$ gleich oder verschieden sind und ein Wasserstoffatom oder einen $C_1$-$C_6$-Alkylrest bedeuten), bedeuten, oder $R^3$ und $R^4$ zusammen genommen einen heterocyclischen Rest bilden, welcher das Stickstoffatom in dem Ring in Sandwich-Stellung enthält, der mit 1 bis 3 Substituenten substituiert sein kann, ausgewählt aus einem $C_1$-$C_6$-Alkylrest und einem

Rest der Formel $NR^7R^8$ (wobei $R^7$ und $R^8$ gleich oder verschieden und ein Wasserstoffatom oder einen $C_1$-$C_6$-Alkylrest bedeuten)}, einem Halogenatom, einer Hydroxygruppe, einem $C_1$-$C_6$-Alkoxy-, $C_1$-$C_6$-Alkoxycarbonyl-, $C_1$-$C_6$-Alkanoyl-, $C_1$-$C_6$-Alknaoyloxyrest und einer Vinylgruppe, oder

(iii) einen Rest der Formel $NR^1R^2$ <wobei $R^1$ und $R^2$ gleich oder verschieden sind und ein Wasserstoffatom, einen $C_1$-$C_6$-Alkylrest bedeuten, der mit 1 bis 3 Substituenten substituiert sein kann, ausgewählt aus einem Rest der Formel $NR^3R^4$ {wobei $R^3$ und $R^4$ gleich oder verschieden sind und ein Wasserstoffatom, einen $C_1$-$C_6$-Alkylrest, der mit einem Rest der Formel $NR^5R^6$ substituiert sein kann, bedeuten (wobei $R^5$ und $R^6$ gleich oder verschieden sind und ein Wasserstoffatom oder einen $C_1$-$C_6$-Alkylrest, bedeuten), oder $R^3$ und $R^4$ zusammen genommen einen heterocyclischen Rest bilden, welcher das Stickstoffatom in dem Ring in Sandwich-Stellung enthält, der mit 1 bis 3 Substituenten substituiert sein kann, ausgewählt aus einem $C_1$-$C_6$-Alkylrest und einem Rest der Formel $NR^7R^8$ (wobei $R^7$ und $R^8$ gleich oder verschieden sind und ein Wasserstoffatom oder einen $C_1$-$C_6$-Alkylrest bedeuten)}, einem Halogenatom, einer Hydroxygruppe, einem $C_1$-$C_6$-Alkoxy-, $C_1$-$C_6$-Alkoxycarbonyl-, $C_1$-$C_6$-Alkanoyl-, $C_1$-$C_6$-Alkanoyloxyrest und einer Vinylgruppe, oder $R^1$ und $R^2$ zusammen genommen einen heterocyclischen Rest bilden, welcher das Stickstoffatom in dem Ring in Sandwich-Stellung enthält, der mit 1 bis 3 Substituenten substituiert sein kann, ausgewählt aus einem $C_1$-$C_6$-Alkylrest und einem Rest der Formel $R^7R^8$ (wobei $R^7$ und $R^8$ gleich oder verschieden sind und ein Wasserstoffatom oder einen $C_1$-$C_6$-Alkylrest bedeuten)>, und

$Y^1$ und $Y^2$ gleich oder verschieden sind und ein Wasserstoffatom, ein Halogenatom oder einen $C_1$-$C_6$-Alkylrest bedeuten,
oder ein pharmazeutisch verträgliches Salz davon.

**2.** 5-Aminoflavon-Derivat nach Anspruch 1, wobei X einen $C_1$-$C_6$-Alkylrest, der mit 1 bis 3 Substituenten substituiert sein kann, ausgewählt aus einem Rest der Formel $NR^3R^4$ {wobei $R^3$ und $R^4$ gleich oder verschieden sind und ein Wasserstoffatom, einen $C_1$-$C_6$-Alkylrest, der mit einem Rest der Formel $NR^5R^6$ substituiert sein kann (wobei $R^5$ und $R^6$ gleich oder verschieden sind und ein Wasserstoffatom oder einen $C_1$-$C_6$-Alkylrest bedeuten), oder $R^3$ und $R^4$ zusammen einen heterocyclischen Rest bilden, welcher das Stickstoffatom in dem Ring in Sandwich-Stellung enthält, der mit 1 bis 3 Substituenten substituiert sein kann, die aus $C_1$-$C_6$-Alkylresten und Resten der Formel $NR^7R^8$ ausgewählt sind (wobei $R^7$ und $R^8$ gleich oder verschieden sind und ein Wasserstoffatom oder einen $C_1$-$C_6$-Alkylrest bedeuten)}, einem Halogenatom, einer Hydroxygruppe, einem $C_1$-$C_6$-Alkoxy-, $C_1$-$C_6$-Alkoxycarbonyl-, $C_1$-$C_6$-Alkanoyl-, $C_1$-$C_6$-Alkanoyloxyrest und einer Vinylgruppe, bedeutet oder ein pharmazeutisch verträgliches Salz davon.

**3.** 5-Aminoflavon-Derivat nach Anspruch 1 oder 2, wobei mindestens einer der Reste $Y^1$ und $Y^2$ ein Halogenatom bedeutet oder ein pharmazeutisch verträgliches Salz davon.

**Revendications**

**1.** Dérivé de 5-aminoflavone, représenté par la formule (1) :

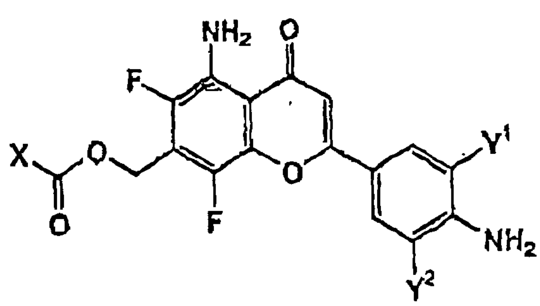

(I)

dans laquelle X représente :

(i) un groupe alkyle en $C_{1-6}$, portant de 1 à 3 substituants, choisis dans l'ensemble que forment les groupes de formule $NR^3R^4$ {où $R^3$ et $R^4$ peuvent être identiques ou différents et représentent chacun un atome d'hydrogène ou un groupe alkyle en $C_{1-6}$, lequel peut porter un substituant de formule $NR^5R^6$ (où $R^5$ et $R^6$ peuvent

être identiques ou différents et représentent chacun un atome d'hydrogène ou un groupe alkyle en $C_{1-6}$), ou encore $R^3$ et $R^4$ constituent ensemble, avec l'atome d'azote qui les relie, un groupe hétérocyclique qui peut porter de 1 à 3 substituants choisis dans l'ensemble que forment les groupes alkyle en $C_{1-6}$ et les groupes de formule $NR^7R^8$ (où $R^7$ et $R^8$ peuvent être identiques ou différents et représentent chacun un atome d'hydrogène ou un groupe alkyle en $C_{1-6}$)}, les atomes d'halogène et les groupes hydroxy, alcoxy en $C_{1-6}$, (alcoxy en $C_{1-6}$)carbonyle, alcanoyle en $C_{1-6}$, alcanoyloxy en $C_{1-6}$ et vinyle,

(ii) un groupe alcoxy en $C_{1-6}$, qui peut porter de 1 à 3 substituants choisis dans l'ensemble que forment les groupes de formule $NR^3R^4$ {où $R^3$ et $R^4$ peuvent être identiques ou différents et représentent chacun un atome d'hydrogène ou un groupe alkyle en $C_{1-6}$, lequel peut porter un substituant de formule $NR^5R^6$ (où $R^5$ et $R^6$ peuvent être identiques ou différents et représentent chacun un atome d'hydrogène ou un groupe alkyle en $C_{1-6}$), ou encore $R^3$ et $R^4$ constituent ensemble, avec l'atome d'azote qui les relie, un groupe hétérocyclique qui peut porter de 1 à 3 substituants choisis dans l'ensemble que forment les groupes alkyle en $C_{1-6}$ et les groupes de formule $NR^7R^8$ (où $R^7$ et $R^8$ peuvent être identiques ou différents et représentent chacun un atome d'hydrogène ou un groupe alkyle en $C_{1-6}$)}, les atomes d'halogène et les groupes hydroxy, alcoxy en $C_{1-6}$, (alcoxy en $C_{1-6}$)carbonyle, alcanoyle en $C_{1-6}$, alcanoyloxy en $C_{1-6}$ et vinyle, ou

(iii) un groupe de formule $NR^1R^2$ < dans laquelle $R^1$ et $R^2$ peuvent être identiques ou différents et représentent chacun un atome d'hydrogène ou un groupe alkyle en $C_{1-6}$ qui peut porter de 1 à 3 substituants choisis dans l'ensemble que forment les groupes de formule $NR^3R^4$ {où $R^3$ et $R^4$ peuvent être identiques ou différents et représentent chacun un atome d'hydrogène ou un groupe alkyle en $C_{1-6}$, lequel peut porter un substituant de formule $NR^5R^6$ (où $R^5$ et $R^6$ peuvent être identiques ou différents et représentent chacun un atome d'hydrogène ou un groupe alkyle en $C_{1-6}$), ou encore $R^3$ et $R^4$ constituent ensemble, avec l'atome d'azote qui les relie, un groupe hétérocyclique qui peut porter de 1 à 3 substituants choisis dans l'ensemble que forment les groupes alkyle en $C_{1-6}$ et les groupes de formule $NR^7R^8$ (où $R^7$ et $R^8$ peuvent être identiques ou différents et représentent chacun un atome d'hydrogène ou un groupe alkyle en $C_{1-6}$)}, les atomes d'halogène et les groupes hydroxy, alcoxy en $C_{1-6}$, (alcoxy en $C_{1-6}$)carbonyle, alcanoyle en $C_{1-6}$, alcanoyloxy en $C_{1-6}$ et vinyle, ou encore $R^1$ et $R^2$ constituent ensemble, avec l'atome d'azote qui les relie, un groupe hétérocyclique qui peut porter de 1 à 3 substituants choisis dans l'ensemble que forment les groupes alkyle en $C_{1-6}$ et les groupes de formule $NR^7R^8$ (où $R^7$ et $R^8$ peuvent être identiques ou différents et représentent chacun un atome d'hydrogène ou un groupe alkyle en $C_{1-6}$) >,

et $Y^1$ et $Y^2$ peuvent être identiques ou différents et représentent chacun un atome d'hydrogène ou d'halogène ou un groupe alkyle en $C_{1-6}$,
ou sel d'un tel dérivé, admissible en pharmacie.

**2.** Dérivé de 5-aminoflavone, conforme à la revendication 1, dans lequel X représente un groupe alkyle en $C_{1-6}$ portant de 1 à 3 substituants choisis dans l'ensemble que forment les groupes de formule $NR^3R^4$ {où $R^3$ et $R^4$ peuvent être identiques ou différents et représentent chacun un atome d'hydrogène ou un groupe alkyle en $C_{1-6}$, lequel peut porter un substituant de formule $NR^5R^6$ (où $R^5$ et $R^6$ peuvent être identiques ou différents et représentent chacun un atome d'hydrogène ou un groupe alkyle en $C_{1-6}$), ou encore $R^3$ et $R^4$ constituent ensemble, avec l'atome d'azote qui les relie, un groupe hétérocyclique qui peut porter de 1 à 3 substituants choisis dans l'ensemble que forment les groupes alkyle en $C_{1-6}$ et les groupes de formule $NR^7R^8$ (où $R^7$ et $R^8$ peuvent être identiques ou différents et représentent chacun un atome d'hydrogène ou un groupe alkyle en $C_{1-6}$)}, les atomes d'halogène et les groupes hydroxy, alcoxy en $C_{1-6}$, (alcoxy en $C_{1-6}$)carbonyle, alcanoyle en $C_{1-6}$, alcanoyloxy en $C_{1-6}$ et vinyle, ou sel d'un tel dérivé, admissible en pharmacie.

**3.** Dérivé de 5-aminoflavone, conforme à la revendication 1 ou 2, dans lequel au moins l'un des symboles $Y^1$ et $Y^2$ représente un atome d'halogène, ou sel d'un tel dérivé, admissible en pharmacie.